# EUROPEAN PATENT APPLICATION

(11) **EP 3 967 698 A1**
(43) Date of publication of application: **16.03.2022**
(21) Application number: 20802767.2
(22) Date of filing: 06.05.2020
(51) Int. Cl.: C07D 519/00, C07D 471/04, A61K 31/5386, A61K 31/5377, A61P 35/00, A61P 35/02

(54) **SALT AND CRYSTAL FORM OF MTORC1/2 DUAL KINASE ACTIVITY INHIBITOR AND PREPARATION METHOD THEREFOR**

(30) Priority: 06.05.2019 CN 201910373071; 08.05.2019 CN 201910380491
(71) Applicant: Medshine Discovery Inc., Nanjing, Jiangsu 210032 (CN)
(72) Inventor: CHEN, Kevin X, Shanghai 200131 (CN); CHEN, Zhaoguo, Shanghai 200131 (CN); ZHANG, Li, Shanghai 200131 (CN); YU, Yanxin, Shanghai 200131 (CN); ZHOU, Kai, Shanghai 200131 (CN); JIANG, Fen, Shanghai 200131 (CN); XIA, Shanghua, Shanghai 200131 (CN); WANG, Xiaofei, Shanghai 200131 (CN)
(74) Representative: Greaves Brewster LLP
(86) International application number: PCT/CN2020/088748
(87) International publication number: WO 2020/224585

(57) **Abstract**

Disclosed are a salt and a crystal form of an mTORC1/2 dual kinase inhibitor and a preparation method therefor. Also disclosed is use of the salt and crystal form in the preparation of a medicament related to the mTORC1/2 dual kinase inhibitor. (I)

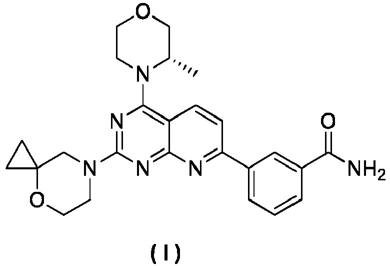

## Description

The present application claims the following priority:
CN201910373071.9, filed on 20190506;
CN201910380491.X, filed on 20190508.

### Technical Field

The present invention relates to a salt and a crystal form of mTORC1/2 dual kinase inhibitor and preparation method thereof, and also includes the use of said salt form and crystal form in the preparation of a medicament related to the mTORC1/2 dual kinase inhibitor.

### Background

Tumor, especially malignant tumor, is one of the most serious diseases endangering human health currently. With the progress of science and technology and the deepening of the research on tumor treatment, rapid progress has been made in tumor occurrence, development mechanism and tumor treatment. Many new mechanisms and biomarkers have been found. The present invention relates to a signal pathway that plays a key role in tumor proliferation, invasion and metastasis and anti-apoptosis, that is, phosphatidylinositol 3 kinase (PI3K)-Akt mammalian rapamycin protease mTOR signal pathway.

The activation of PI3K largely comes from the substrate near the inner side of its plasma membrane. The activation of PI3K can be initiated by a variety of growth factors and signal transduction complexes, including fibroblast growth factor (FGF), vascular endothelial growth factor (VEGF), human growth factor (HGF), vascular position protein I (Ang1) and insulin. These factors activate receptor tyrosine kinase (RTK), resulting in autophosphorylation. As the result of PI3K activation, on the plasma membrane is produced the second messenger PIP3 that binds to the signal proteins Akt and PDK1 (phosphoinoside dependent kinase-1) containing PH domain in the cell, prompting PDK1 to phosphorylate ser308 of Akt protein, resulting in Akt activation. Other substrates of PDK1 also include PKC (protein kinase C), S6K (p70S6) and SGK (serum/ glucocorticoid regulated kinases). Akt, also known as protein kinase B (PKB), is the main effector downstream of PI3K. The activated Akt regulates cell function by phosphorylating downstream factors such as enzymes, kinases and transcription factors. Akt plays an anti-apoptotic role by phosphorylating target proteins in multiple downstream pathways. PTEN (phosphotase and tensin homology deleted on chromosome 10) is a tumor suppressor gene, which has been mutated or deleted in a wide range of human tumors. PTEN is a PIP3 phosphatase that can convert PIP3 to PIP2 by dephosphorylation, which is contrary to the function of PI3K. PTEN can reduce the activation of Akt and thus prevent all downstream signal transduction events regulated by Akt. mTOR, as the downstream substrate of Akt, is relatively conservative in evolution, can integrate a variety of signals of nutrition, energy and growth factors, participate in biological processes such as gene transcription, protein translation, ribosome synthesis and apoptosis, and therefore play a very important role in cell growth. There are two highly homologous complexes, in which Tor binds with KOG01 to form mTORC1, and mTOR binds with AVO1/AVO2/AVO3/LST8 to form mTORC2 insensitive to rapamycin. mTOR regulates downstream protein translation by phosphorylating downstream target proteins S40S ribosomal S6 protein kinase, such as S6K1 and 4EBP1. mTOR binding to eIF3 phosphorylates S6K1, resulting in S6K1 released from eIF3 and activated, further phosphorylates cell substrates, such as p70S6, and promotes protein translation and expression. 4EBP1 binds to eukaryotic transcription promoter 4E and inhibits its activity. After phosphorylation of 4E-BP1 by mTOR, 4E-BP1 is activated for separation from eIF-4E to realize eukaryotic transcription. mTORC2 can phosphorylate Akt and thus up-regulate its kinase activity.

It can be seen from the above that any mutation or overexpression in the upstream of PI3K/ Akt/ mTOR signaling pathway will lead to a series of cascade reactions in the downstream, finally leading to the occurrence, development and metastasis of tumor. mTOR is a key position in signal pathway, in which the inhibition of mTORC1 and mTORC2 can block the transmission of signal, so as to control the development of tumor.

It is found that this signaling pathway is involved in many solid tumors, such as breast cancer, prostate cancer, lung cancer, colon cancer, pancreatic cancer, liver cancer, gastric cancer, colorectal cancer, renal carcinoma, thyroid cancer, meningitis cancer, acute and chronic lymphocytic leukemia, Meckel cell tumor and so on, and is closely related to therapeutic tolerance and poor prognosis. It can be seen that a good development prospect can be achieved by the development of small molecular compounds to inhibit the signal pathway of PI3K/ Akt/ mTOR. The present invention aims to find a targeted medicament of di-mTOR small molecule compound, which has good activity and shows excellent effect and function.

US20170281637 disclosed compound AZD2014, belonging to mTORC1& mTORC2 kinase inhibitor, which has the following structural formula:

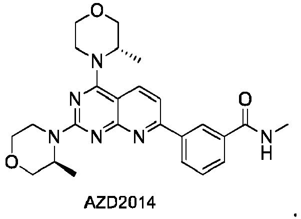

### Summary of the Invention

The present invention also provides a crystal form A of the compound of formula (I), wherein the X-ray powder diffraction pattern thereof has characteristic diffraction peaks at the following 2θ angles: 16.355 ± 0.200° 19.847 ± 0.200°, and 21.319 ± 0.200°,

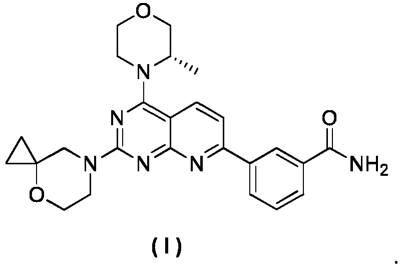

In some embodiments of the present invention, the X-ray powder diffraction pattern of the crystal form A of the compound of formula (I) has characteristic diffraction peaks at the following 2θ angles: 8.169 ±0.200°, 10.986 ±0.200°, 13.394 ± 0.200°, 16.355 ±0.200°, 17.084 ±0.200°, 19.847 ±0.200°, 21.319 ±0.200°, and 22.154 ± 0.200°.

In some embodiments of the present invention, the X-ray powder diffraction pattern of the crystal form A of the compound of formula (I) has characteristic diffraction peaks at the following 2θ angles: 8.169 ± 0.200°, 9.903 ± 0.200°, 10.986 ± 0.200°, 13.394 ±0.200° 13.931 ±0.200° 15.330 ±0.200° 16.355 ±0.200° 17.084 ± 0.200° 19.847 ± 0.200°, 21.319 ± 0.200°, 22.154 ± 0.200° and 23.475 ±0.200°.

In some embodiments of the present invention, the X-ray powder diffraction pattern of the crystal form A of the compound of formula (I) has characteristic diffraction peaks at the following 2θ angles: 8.169°, 9.119°, 9.551°, 9.903°, 10.986°, 12.525°, 13.394°, 13.931°, 15.330°, 15.589°, 15.923°, 16.355°, 16.743°, 17.084°, 17.815°, 18.268°, 19.149°, 19.414°, 19.847°, 20.273°, 21.319°, 22.154°, 23.475°, 23.953°, 24.710 °, 25.134 °, 25.805°, 26.054 °, 26.232 °, 26.772 °, 28.048 °, 29.254 °, 29.469°, 29.958 °, 31.617 °, 31.838 °, 33.074°, 33.924 °, 34.281 °, 36.061°, 36.520°, and 38.052°.

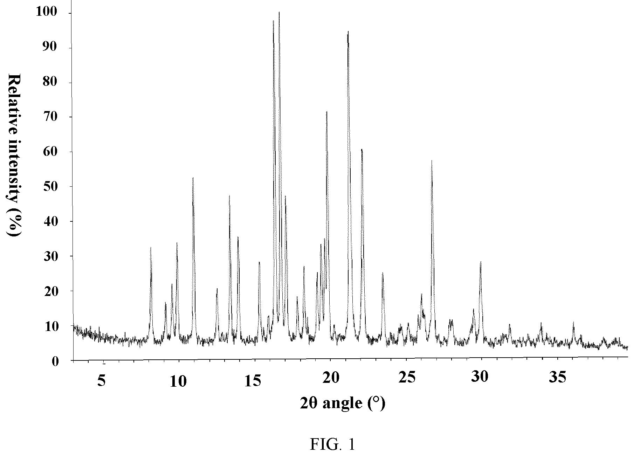

In some embodiments of the present invention, the X-ray powder diffraction pattern of the crystal form A of the compound of formula (I) is shown in Fig. 1.

In some embodiments of the present invention, the analytical data of the X-ray powder diffraction pattern of the crystal form A of the compound of formula (I) are shown in Table 1:

**Table 1**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 8.169 | 10.8142 | 29.0 | 22 | 22.154 | 4.0092 | 57.6 |
| 2 | 9.119 | 9.6899 | 11.9 | 23 | 23.475 | 3.7864 | 21.4 |
| 3 | 9.551 | 9.2524 | 17.5 | 24 | 23.953 | 3.7121 | 3.1 |
| 4 | 9.903 | 8.9239 | 29.6 | 25 | 24.710 | 3.5999 | 4.4 |
| 5 | 10.986 | 8.0470 | 50.2 | 26 | 25.134 | 3.5402 | 5.4 |
| 6 | 12.525 | 7.0616 | 15.5 | 27 | 25.805 | 3.4497 | 7.6 |
| 7 | 13.394 | 6.6052 | 43.4 | 28 | 26.054 | 3.4172 | 13.8 |
| 8 | 13.931 | 6.3518 | 31.4 | 29 | 26.232 | 3.3945 | 7.8 |
| 9 | 15.330 | 5.7752 | 23.9 | 30 | 26.772 | 3.3272 | 55.1 |
| 10 | 15.589 | 5.6797 | 3.7 | 31 | 28.048 | 3.1787 | 6.8 |
| 11 | 15.923 | 5.5612 | 5.6 | 32 | 29.254 | 3.0503 | 3.7 |
| 12 | 16.355 | 5.4153 | 97.4 | 33 | 29.469 | 3.0285 | 10.4 |
| 13 | 16.743 | 5.2906 | 100.0 | 34 | 29.958 | 2.9802 | 25.2 |
| 14 | 17.084 | 5.1858 | 43.2 | 35 | 31.617 | 2.8275 | 3.1 |
| 15 | 17.815 | 4.9746 | 12.7 | 36 | 31.838 | 2.8084 | 5.8 |
| 16 | 18.268 | 4.8524 | 21.7 | 37 | 33.074 | 2.7062 | 3.3 |
| 17 | 19.149 | 4.6310 | 19.5 | 38 | 33.924 | 2.6403 | 6.6 |
| 18 | 19.414 | 4.5685 | 28.7 | 39 | 34.281 | 2.6136 | 3.7 |
| 19 | 19.847 | 4.4698 | 69.8 | 40 | 36.061 | 2.4886 | 7.3 |
| 20 | 20.273 | 4.3768 | 3.8 | 41 | 36.520 | 2.4584 | 3.4 |
| 21 | 21.319 | 4.1642 | 93.8 | 42 | 38.052 | 2.3628 | 3.2 |

.

In some embodiments of the present invention, the differential scanning calorimetry curve of the crystal form A of formula (I) has an endothermic peak with onset at 219.38 ±3.00 °C.

In some embodiments of the present invention, the differential scanning calorimetry curve of the crystal form A of formula (I) has an endothermic peak at 221.62 ± 3.00 °C.

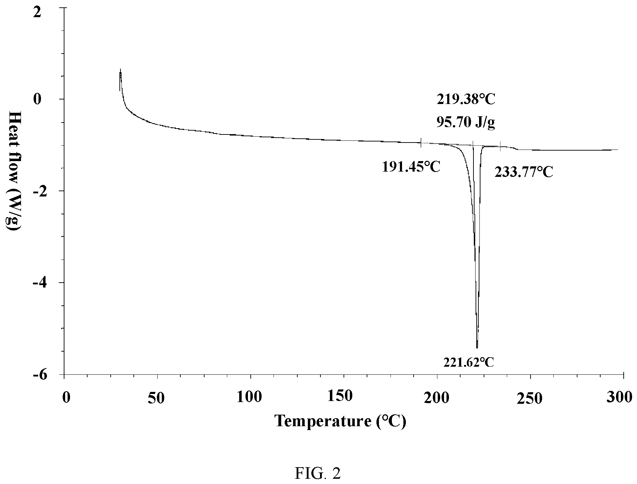

In some embodiments of the present invention, the DSC pattern of the crystal form A of formula (I) is shown in Fig. 2.

In some embodiments of the present invention, the thermal gravimetric analysis curve of the crystal form A of formula (I) has a weight loss of 0.08225% at 122.98 ±3.00 °C, and a further weight loss of 0.4156% at 262.62 ±3.00 °C.

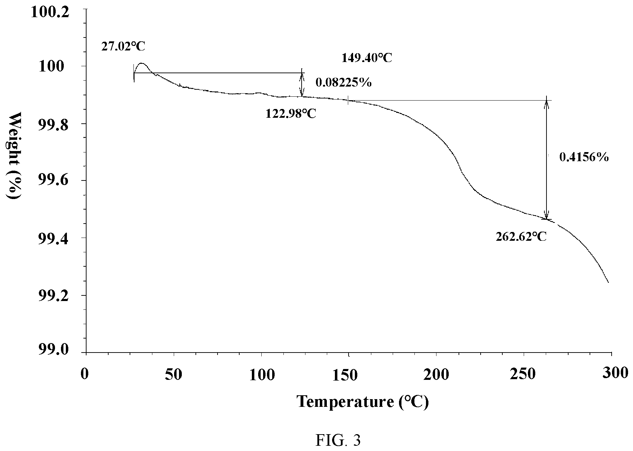

In some embodiments of the present invention, the TGA pattern of the crystal form A of formula (I) is shown in Fig. 3.

The present invention also provides a crystal form B of the compound of formula (I), wherein the X-ray powder diffraction pattern thereof has characteristic diffraction peaks at the following 2θ angles: 6.295 ± 0.200°, 7.851 ± 0.200°, and 11.324 ± 0.200°, 16.351 ± 0.200°,

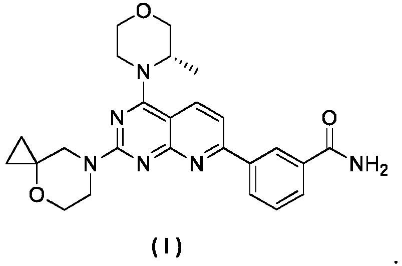

In some embodiments of the present invention, the X-ray powder diffraction pattern of the crystal form B of the compound of formula (I) has characteristic diffraction peaks at the following 2θ angles: 6.295 ± 0.200°, 7.851 ± 0.200°, 8.621 ± 0.100°, 11.324 ±0.100°, 14.184 ±0.100°, 15.287 ±0.200°, 16.351 ±0.200°, and 26.114 ±0.200°.

In some embodiments of the present invention, the X-ray powder diffraction pattern of the crystal form B of the compound of formula (I) has characteristic diffraction peaks at the following 2θ angles: 6.295 ±0.200°, 7.851 ±0.200°, 8.621 ± 0.200°, 11.324 ±0.200°, 14.184 ±0.200°, 15.287 ±0.200°, 16.351 ±0.200°, and 26.114 ±0.200°.

In some embodiments of the present invention, the X-ray powder diffraction pattern of the crystal form B of the compound of formula (I) has characteristic diffraction peaks at the following 2θ angles: 6.295 ±0.200°, 7.851 ±0.200°. 8.621 ± 0.100°, 11.324 ±0.100° 14.184 ±0.100° 15.287 ±0.200° 16.351 ±0.200° 17.087 ± 0.200°, 18.563 ±0.200°, 19.531 ±0.200°, 20.791 ±0.200°, and 26.114 ±0.200°.

In some embodiments of the present invention, the X-ray powder diffraction pattern of the crystal form B of the compound of formula (I) has characteristic diffraction peaks at the following 2θ angles: 6.295 ±0.200°, 7.851 ±0.200°, 8.621 ± 0.200°, 11.324 ±0.200° 14.184 ±0.200°, 15.287 ±0.200° 16.351 ±0.200° 17.087 ± 0.200° 18.563 ±0.200°, 19.531 ±0.200°, 20.791 ±0.200°, and 26.114 ±0.200°.

In some embodiments of the present invention, the X-ray powder diffraction pattern of the crystal form B of the compound of formula (I) has characteristic diffraction peaks at the following 2θ angles: 6.295° 7.851°, 8.166°, 8.621°, 9.109°, 9.528°, 9.897°, 10.985°, 11.324°, 11.973°, 12.586°, 12.876°, 13.391°, 13.928°, 14.184° 15.287°, 15.879° 16.351°, 16.590°, 17.087°, 17.276°, 18.563°, 18.933°, 19.531°, 19.827°, 20.791°, 21.285°, 22.136, 22.585°, 23.489°, 23.650°, 24.151° 24.970° 25.328° 25.900°, 26.114°, 26.731°, 28.109°, 28.659°, 29.551°, 29.908°, 31.539°, 36.554°, and 38.644°.

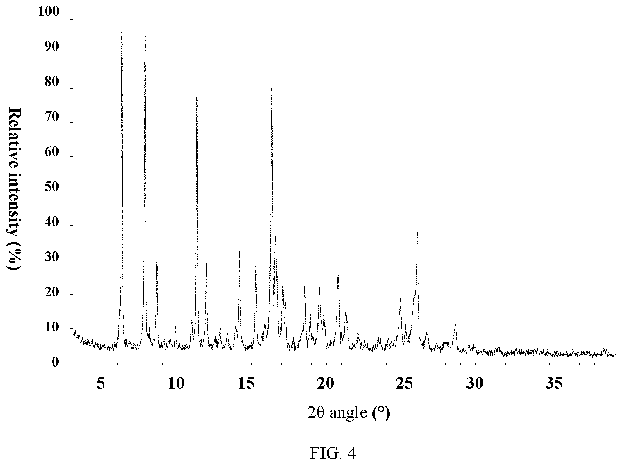

In some embodiments of the present invention, the X-ray powder diffraction pattern of the crystal form B of the compound of formula (I) is shown in Fig. 4.

In some embodiments of the present invention, the analytical data of the X-ray powder diffraction pattern of the crystal form B of the compound of formula (I) are shown in Table 2,

**Table 2**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 6.295 | 14.0299 | 96.7 | 23 | 18.933 | 4.6833 | 8.7 |
| 2 | 7.851 | 11.2513 | 100.0 | 24 | 19.531 | 4.5413 | 17.5 |
| 3 | 8.166 | 10.8177 | 6.0 | 25 | 19.827 | 4.4742 | 9.3 |
| 4 | 8.621 | 10.2485 | 26.1 | 26 | 20.791 | 4.2689 | 22.0 |
| 5 | 9.109 | 9.6999 | 2.6 | 27 | 21.285 | 4.1710 | 10.8 |
| 6 | 9.528 | 9.2751 | 3.1 | 28 | 22.136 | 4.0125 | 6.5 |
| 7 | 9.897 | 8.9298 | 6.9 | 29 | 22.585 | 3.9336 | 3.0 |
| 8 | 10.985 | 8.0480 | 9.4 | 30 | 23.489 | 3.7843 | 3.9 |
| 9 | 11.324 | 7.8073 | 80.5 | 31 | 23.650 | 3.7588 | 4.1 |
| 10 | 11.973 | 7.3859 | 25.1 | 32 | 24.151 | 3.6820 | 2.7 |
| 11 | 12.586 | 7.0271 | 3.4 | 33 | 24.970 | 3.5631 | 12.7 |
| 12 | 12.876 | 6.8696 | 5.9 | 34 | 25.328 | 3.5135 | 6.1 |
| 13 | 13.391 | 6.6064 | 4.9 | 35 | 25.900 | 3.4372 | 14.4 |
| 14 | 13.928 | 6.3532 | 6.7 | 36 | 26.114 | 3.4095 | 34.6 |
| 15 | 14.184 | 6.2388 | 30.0 | 37 | 26.731 | 3.3322 | 4.7 |
| 16 | 15.287 | 5.7913 | 24.9 | 38 | 28.109 | 3.1719 | 2.6 |
| 17 | 15.879 | 5.5768 | 4.3 | 39 | 28.659 | 3.1122 | 7.7 |
| 18 | 16.351 | 5.4167 | 79.3 | 40 | 29.551 | 3.0203 | 2.2 |
| 19 | 16.59 | 5.3390 | 33.1 | 41 | 29.908 | 2.9851 | 2.4 |
| 20 | 17.087 | 5.1849 | 15.8 | 42 | 31.539 | 2.8343 | 2.4 |
| 21 | 17.276 | 5.1287 | 12.3 | 43 | 36.554 | 2.4562 | 1.8 |
| 22 | 18.563 | 4.7760 | 17.4 | 44 | 38.644 | 2.3280 | 2.6 |

The present invention also provides a compound of formula (II),

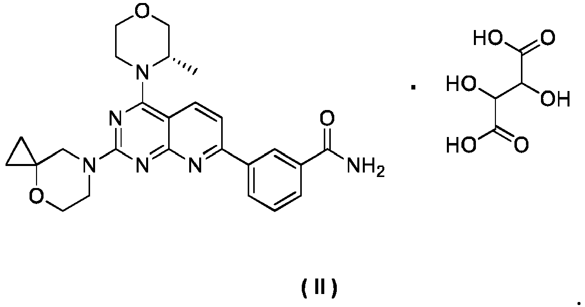

The present invention also provides a crystal form C of the compound of formula (II), wherein the X-ray powder diffraction pattern thereof has characteristic diffraction peaks at the following 2θ angles: 5.802 ± 0.200°, 7.991 ± 0.200°, 11.618 ± 0.200°, and 16.567 ±0.200°.

In some embodiments of the present invention, the X-ray powder diffraction pattern of the crystal form C of the compound of formula (II) has characteristic diffraction peaks at the following 2θ angles: 5.802 ± 0.200°, 7.991 ± 0.200°, 11.618 ± 0.200°, 13.138 ±0.100°, 15.487 ±0.100°, 15.959 ±0.200 °, 16.567 ±0.200°, and 18.268 ± 0.200°.

In some embodiments of the present invention, the X-ray powder diffraction pattern of the crystal form C of the compound of formula (II) has characteristic diffraction peaks at the following 2θ angles: 5.802 ±0.200°, 7.991 ±0.200°, 11.618 ± 0.200 °, 13.138 ±0.200 °, 15.487 ±0.200°, 15.959 ±0.200°, 16.567 ±0.200°, and 18.268 ±0.200°.

In some embodiments of the present invention, the X-ray powder diffraction pattern of the crystal form C of the compound of formula (II) has characteristic diffraction peaks at the following 2θ angles: 5.802 ±0.200°, 7.991 ±0.200°, 11.618 ± 0.200°, 13.138 ±0.100°, 15.487 ±0.100°, 15.959 ±0.200° 16.567 ±0.200°, 18.268 ± 0.200° 18.802 ±0.200° 19.505 ±0.200° 22.309 ±0.200° and 23.633 ±0.200°.

In some embodiments of the present invention, the X-ray powder diffraction pattern of the crystal form C of the compound of formula (II) has characteristic diffraction peaks at the following 2θ angles: 5.802 ±0.200°, 7.991 ±0.200°, 11.618 ± 0.200°, 13.138 ±0.200° 15.487 ±0.200° 15.959 ±0.200° 16.567 ±0.200° 18.268 ± 0.200°, 18.802 ±0.200°, 19.505 ±0.200°, 22.309 ±0.200°, and 23.633 ±0.200°.

In some embodiments of the present invention, the X-ray powder diffraction pattern of the crystal form C of the compound of formula (II) has characteristic diffraction peaks at the following 2θ angles: 5.802°, 7.991°, 11.127°, 11.618°, 13.138°, 14.219°, 14.637°, 15.487 °, 15.959°, 16.567°, 18.268°, 18.802°, 19.098°, 19.505°, 19.881°, 20.343°, 21.061°, 21.340°, 21.977°, 22.309°, 22.504°, 23.298°, 23.633°, 24.403°, 24.954°, 25.286°, 25.557°, 26.176°, 26.986°, 28.618°, 29.472°, 29.805°, 31.796°, 32.052°, 32.666°, 33.433°, 34.423°, 34.836°, 38.939°, and 39.312 °.

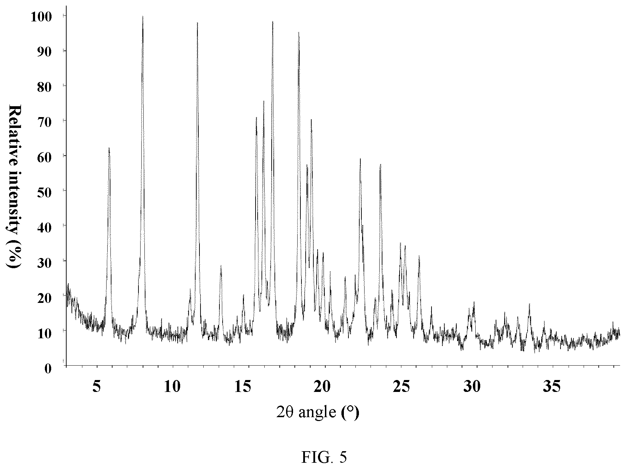

In some embodiments of the present invention, the X-ray powder diffraction pattern of the crystal form C of the compound of formula (II) is shown in Fig. 5.

In some embodiments of the present invention, the analytical data of the X-ray powder diffraction pattern of the crystal form C of the compound of formula (II) are shown in Table 3:

**Table 3**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 5.802 | 15.2205 | 56.8 | 21 | 22.504 | 3.9477 | 28.1 |
| 2 | 7.991 | 11.0552 | 100.0 | 22 | 23.298 | 3.8148 | 11.4 |
| 3 | 11.127 | 7.9451 | 14.5 | 23 | 23.633 | 3.7616 | 52.8 |
| 4 | 11.618 | 7.6105 | 98.5 | 24 | 24.403 | 3.6446 | 12.3 |
| 5 | 13.138 | 6.7331 | 22.2 | 25 | 24.954 | 3.5653 | 27.4 |
| 6 | 14.219 | 6.2238 | 5.9 | 26 | 25.286 | 3.5193 | 26.7 |
| 7 | 14.637 | 6.0469 | 11.6 | 27 | 25.557 | 3.4825 | 13.2 |
| 8 | 15.487 | 5.7169 | 62.1 | 28 | 26.176 | 3.4015 | 24.6 |
| 9 | 15.959 | 5.5489 | 71.5 | 29 | 26.986 | 3.3013 | 9.6 |
| 10 | 16.567 | 5.3465 | 93.4 | 30 | 28.618 | 3.1167 | 6.3 |
| 11 | 18.268 | 4.8523 | 90.1 | 31 | 29.472 | 3.0282 | 11.2 |
| 12 | 18.802 | 4.7157 | 49.1 | 32 | 29.805 | 2.9952 | 12.9 |
| 13 | 19.098 | 4.6433 | 62.1 | 33 | 31.796 | 2.812 | 9.4 |
| 14 | 19.505 | 4.5473 | 22.1 | 34 | 32.052 | 2.7901 | 5.7 |
| 15 | 19.881 | 4.4622 | 20.6 | 35 | 32.666 | 2.739 | 8.3 |
| 16 | 20.343 | 4.3618 | 17.5 | 36 | 33.433 | 2.678 | 12.9 |
| 17 | 21.061 | 4.2146 | 5.5 | 37 | 34.423 | 2.6032 | 7 |
| 18 | 21.340 | 4.1603 | 17.1 | 38 | 34.836 | 2.5732 | 5 |
| 19 | 21.977 | 4.0411 | 18 | 39 | 38.939 | 2.311 | 7.4 |
| 20 | 22.309 | 3.9817 | 55.1 | 40 | 39.312 | 2.29 | 5.9 |

In some embodiments of the present invention, the differential scanning calorimetry curve of the crystal form C of the compound of formula (II) has an endothermic peak with onset at 226.90 ±3.00 °C.

In some embodiments of the present invention, the differential scanning calorimetry curve of the crystal form C of the compound of formula (II) has an endothermic peak at 229.91 ±3.00 °C.

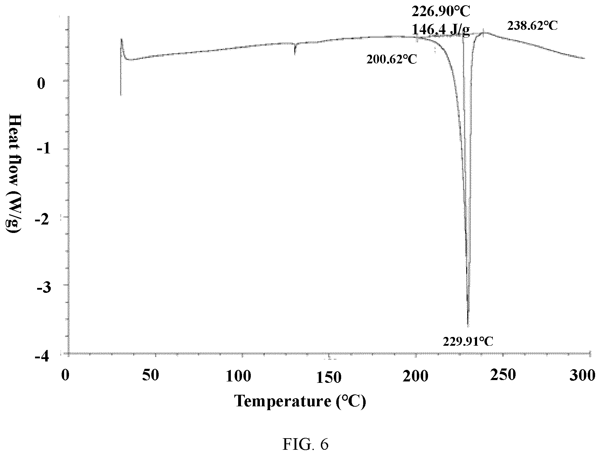

In some embodiments of the present invention, the DSC pattern of the crystal form C of the compound of formula (II) is shown in Fig. 6.

In some embodiments of the present invention, the thermal gravimetric analysis curve of the crystal form C of the compound of formula (II) has a weight loss of 0.1862% at 143.66 ±3.00 °C.

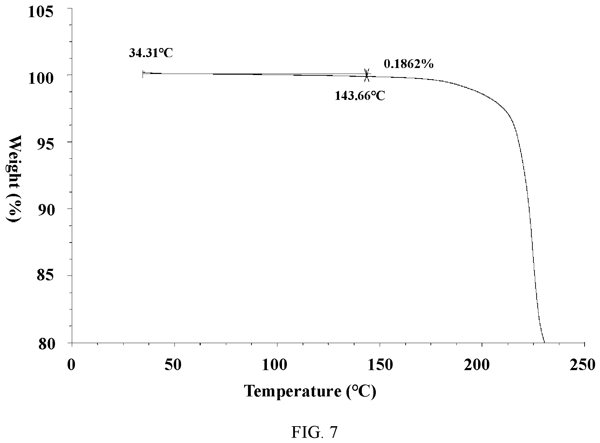

In some embodiments of the present invention, the TGA pattern of the crystal form C of formula (II) is shown in Fig. 7.

The present invention also provides a method for preparing the crystal form A of compound of formula (I), including:
(a) adding the compound of formula (I) to a solvent;
(b) stirring at 30-50 °C for 45-55 hours;
(c) obtaining a solid by centrifugation as the crystal form A of the compound of formula (I);
wherein said solvent is water, acetone, acetonitrile, tetrahydrofuran, methyl t-butyl ether or ethyl acetate.

The present invention also provides a compound of formula (III),

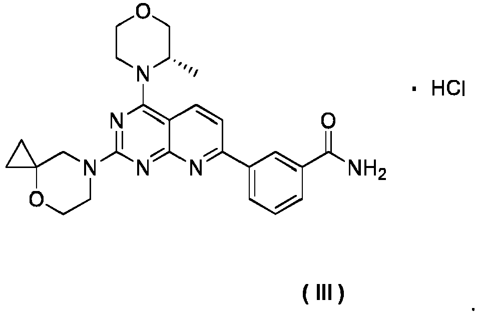

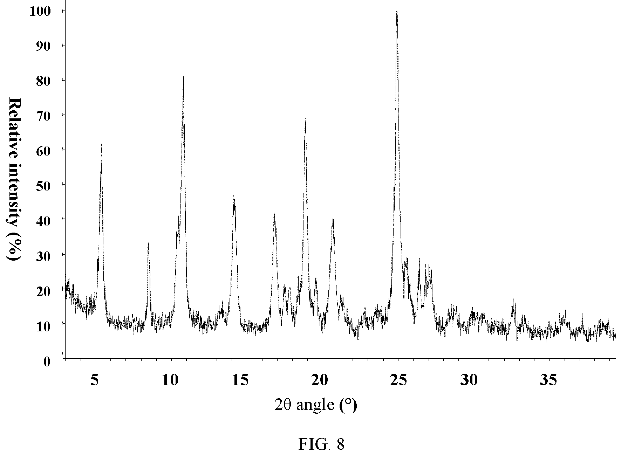

The present invention also provides a crystal form D of the compound of formula (III), wherein the X-ray powder diffraction pattern thereof is shown in Fig. 8.

In some embodiments of the present invention, the analytical data of the XRPD pattern of the crystal form D of the compound of formula (III) are shown in Table 4:

**Table 4**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 5.367 | 16.4519 | 57.6 | 11 | 20.672 | 4.2932 | 33.7 |
| 2 | 8.503 | 10.3905 | 26.4 | 12 | 22.793 | 3.8982 | 6.2 |
| 3 | 10.339 | 8.5486 | 29.3 | 13 | 24.914 | 3.5709 | 100 |
| 4 | 10.789 | 8.1932 | 81.2 | 14 | 25.526 | 3.4867 | 10.4 |
| 5 | 14.125 | 6.2651 | 41.7 | 15 | 26.391 | 3.3743 | 18.6 |
| 6 | 16.808 | 5.2704 | 35.7 | 16 | 26.811 | 3.3225 | 18.4 |
| 7 | 17.498 | 5.0641 | 10.6 | 17 | 27.197 | 3.2761 | 17.5 |
| 8 | 17.795 | 4.9802 | 9.5 | 18 | 28.862 | 3.0908 | 6.7 |
| 9 | 18.859 | 4.7016 | 66.5 | 19 | 32.737 | 2.7333 | 8.6 |
| 10 | 19.547 | 4.5376 | 11.3 | | | | |

The present invention also provides a compound of formula (IV),

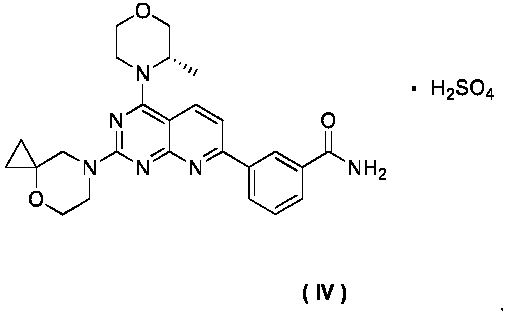

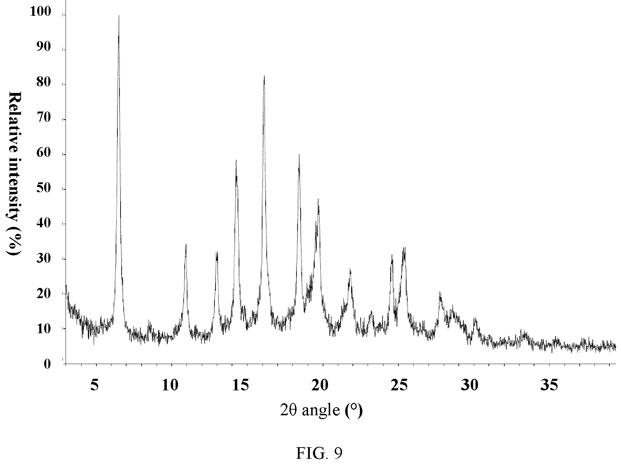

The present invention also provides a crystal form E of the compound of formula (IV), wherein the X-ray powder diffraction pattern thereof is shown in Fig. 9.

In some embodiments of the present invention, the analytical data of the XRPD pattern of crystal form E are shown in Table 5:

**Table 5**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 6.509 | 13.569 | 100.0 | 10 | 19.688 | 4.5054 | 37.7 |
| 2 | 8.574 | 10.3045 | 4.6 | 11 | 21.799 | 4.0736 | 19.7 |
| 3 | 10.947 | 8.0758 | 29.8 | 12 | 23.22 | 3.8276 | 6 |
| 4 | 13.018 | 6.7951 | 25 | 13 | 24.578 | 3.619 | 21 |
| 5 | 14.263 | 6.2044 | 52.8 | 14 | 25.444 | 3.4977 | 23.6 |
| 6 | 14.798 | 5.9816 | 6.3 | 15 | 27.812 | 3.205 | 11.2 |
| 7 | 16.133 | 5.4895 | 79.2 | 16 | 28.602 | 3.1184 | 7 |
| 8 | 18.444 | 4.8065 | 50.1 | 17 | 30.064 | 2.9699 | 6.8 |
| 9 | 18.956 | 4.6778 | 7.6 | | | | |

The present invention also provides a compound of formula (V),

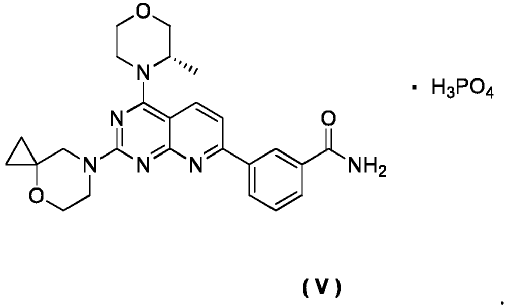

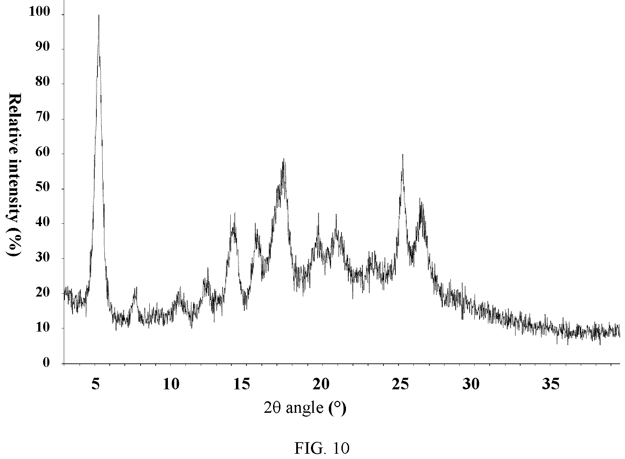

The present invention also provides a crystal form F of the compound of formula (V), wherein the X-ray powder diffraction pattern thereof is shown in Fig. 10.

In some embodiments of the present invention, the analytical data of the XRPD pattern of the crystal form F of formula (V) are shown in Table 6:

**Table 6**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 5.286 | 16.7028 | 100 | 9 | 17.438 | 5.0814 | 37.4 |
| 2 | 7.769 | 11.3708 | 11.2 | 10 | 19.515 | 4.545 | 9.6 |
| 3 | 10.647 | 8.3024 | 10.3 | 11 | 19.735 | 4.4949 | 18.5 |
| 4 | 12.176 | 7.2631 | 9.8 | 12 | 20.910 | 4.2448 | 17.8 |
| 5 | 12.275 | 7.2048 | 10 | 13 | 21.325 | 4.1632 | 10.7 |
| 6 | 14.224 | 6.2215 | 29 | 14 | 25.288 | 3.519 | 36.2 |
| 7 | 15.702 | 5.6389 | 15.2 | 15 | 26.615 | 3.3465 | 20.1 |
| 8 | 17.004 | 5.2102 | 25.5 | | | | |

The present invention also provides a compound of formula (VI),

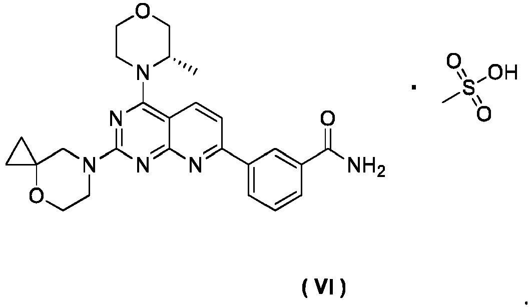

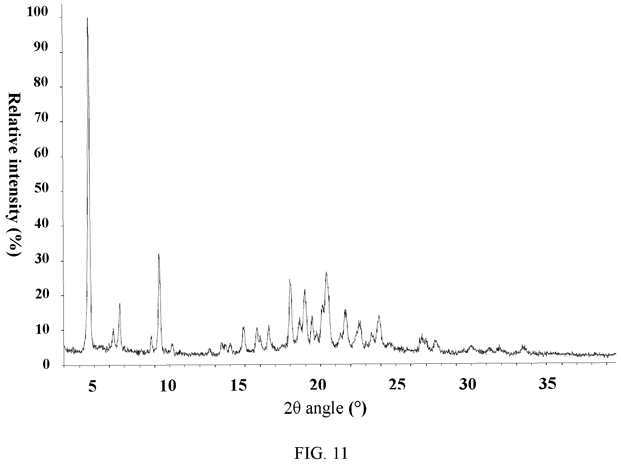

The present invention also provides a crystal form G of the compound of formula (VI), wherein the X-ray powder diffraction pattern thereof is shown in Fig. 11.

In some embodiments of the present invention, the analytical data of the XRPD pattern of the crystal form G of formula (VI) are shown in Table 7:

**Table 7**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 4.677 | 18.8765 | 100 | 18 | 18.994 | 4.6685 | 15.3 |
| 2 | 6.293 | 14.0336 | 6.5 | 19 | 19.471 | 4.5551 | 7.2 |
| 3 | 6.710 | 13.1618 | 14.2 | 20 | 19.745 | 4.4925 | 2.5 |
| 4 | 8.812 | 10.0271 | 5.1 | 21 | 20.178 | 4.3971 | 11.5 |
| 5 | 9.316 | 9.4853 | 30 | 22 | 20.437 | 4.3421 | 21.1 |
| 6 | 10.16 | 8.699 | 2.8 | 23 | 21.346 | 4.1591 | 3.9 |
| 7 | 10.671 | 8.2834 | 1.4 | 24 | 21.697 | 4.0925 | 10.8 |
| 8 | 12.638 | 6.9982 | 2 | 25 | 22.645 | 3.9235 | 7.7 |
| 9 | 13.469 | 6.5684 | 3.6 | 26 | 23.395 | 3.7992 | 4.3 |
| 10 | 13.670 | 6.4722 | 3 | 27 | 23.890 | 3.7217 | 9.8 |
| 11 | 14.025 | 6.3094 | 3.1 | 28 | 26.746 | 3.3304 | 4.8 |
| 12 | 14.952 | 5.9203 | 7.7 | 29 | 27.004 | 3.2991 | 3.8 |
| 13 | 15.821 | 5.597 | 6.9 | 30 | 27.614 | 3.2276 | 2.8 |
| 14 | 16.053 | 5.5164 | 3.6 | 31 | 30.040 | 2.9723 | 2.4 |
| 15 | 16.608 | 5.3335 | 7.5 | 32 | 31.204 | 2.864 | 1.4 |
| 16 | 17.992 | 4.9261 | 19.4 | 33 | 31.856 | 2.8068 | 2.6 |
| 17 | 18.641 | 4.756 | 7.5 | 34 | 33.397 | 2.6808 | 2.9 |

The present invention also provides a compound of formula (VII),

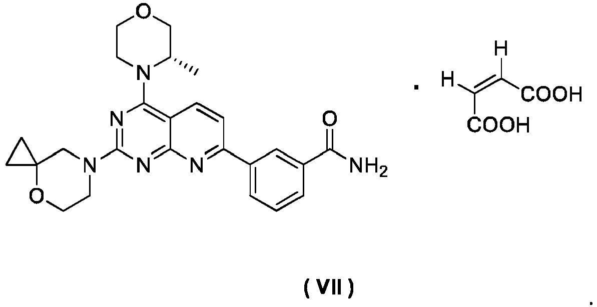

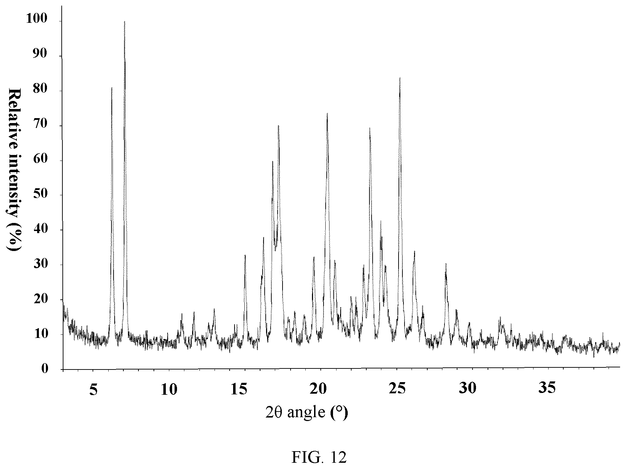

The invention also provides a crystal form H of the compound of formula (VII), wherein the X-ray powder diffraction pattern thereof is as shown in Fig. 12.

In some embodiments of the present invention, the analytical data of the XRPD pattern of the crystal form H of formula (VII) are shown in Table 8:

**Table 8**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 6.296 | 14.0269 | 79.3 | 18 | 22.33 | 3.978 | 10.7 |
| 2 | 7.160 | 12.3356 | 100 | 19 | 22.860 | 3.887 | 21.4 |
| 3 | 10.847 | 8.1495 | 9.5 | 20 | 23.299 | 3.8147 | 64.1 |
| 4 | 11.637 | 7.5979 | 10 | 21 | 23.985 | 3.7071 | 35.1 |
| 5 | 12.606 | 7.016 | 6.4 | 22 | 24.266 | 3.6649 | 21.2 |
| 6 | 12.98 | 6.8147 | 10.7 | 23 | 25.286 | 3.5192 | 80.7 |
| 7 | 14.466 | 6.1178 | 6.4 | 24 | 26.196 | 3.399 | 27.1 |
| 8 | 15.036 | 5.8874 | 27.3 | 25 | 26.716 | 3.334 | 9.5 |
| 9 | 16.255 | 5.4484 | 31.8 | 26 | 28.285 | 3.1526 | 24.5 |
| 10 | 16.887 | 5.246 | 54.6 | 27 | 28.977 | 3.0789 | 9.5 |
| 11 | 17.297 | 5.1224 | 66 | 28 | 29.781 | 2.9975 | 6.6 |
| 12 | 18.291 | 4.8463 | 8.1 | 29 | 30.588 | 2.9202 | 5.2 |
| 13 | 18.918 | 4.6871 | 8.1 | 30 | 31.837 | 2.8085 | 8.5 |
| 14 | 19.565 | 4.5334 | 25.4 | 31 | 32.049 | 2.7903 | 6.4 |
| 15 | 20.495 | 4.3299 | 68.7 | 32 | 32.548 | 2.7488 | 5.9 |
| 16 | 20.932 | 4.2404 | 22.1 | 33 | 36.173 | 2.4812 | 4.7 |
| 17 | 22.016 | 4.034 | 9.5 | 34 | 38.604 | 2.3303 | 5.5 |

The present invention also provides a compound of formula (VIII),

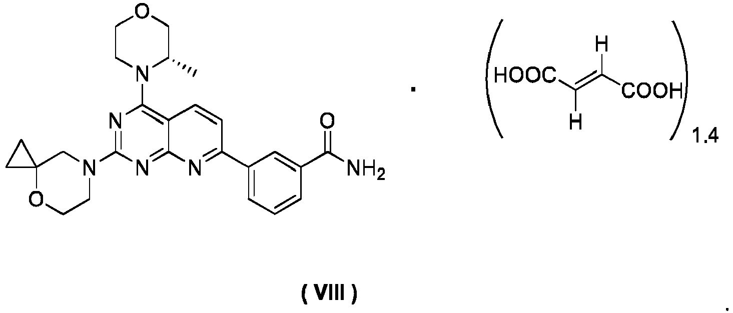

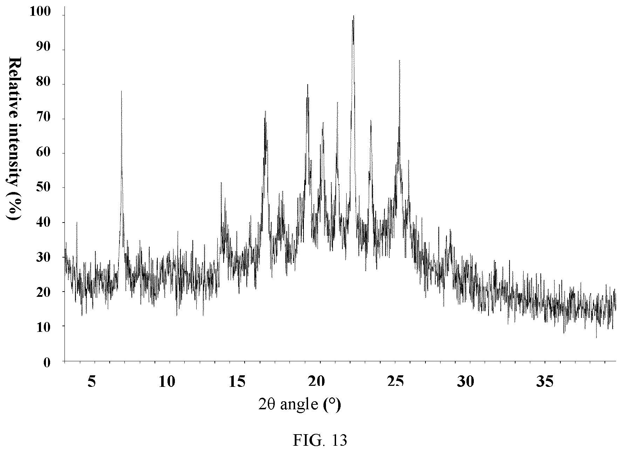

The present invention also provides a crystal form I of the compound of formula (VIII), wherein the X-ray powder diffraction pattern thereof is shown in Fig. 13.

In some embodiments of the present invention, the analytical data of the XRPD pattern of the crystal form I of formula (VIII) are shown in Table 9:

**Table 9**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 6.788 | 13.0109 | 85 | 8 | 20.198 | 4.3929 | 44 |
| 2 | 13.439 | 6.5833 | 42 | 9 | 21.144 | 4.1983 | 60 |
| 3 | 13.69 | 6.4629 | 34 | 10 | 22.248 | 3.9924 | 100 |
| 4 | 16.432 | 5.39 | 60 | 11 | 23.378 | 3.8019 | 55 |
| 5 | 17.300 | 5.1217 | 27 | 12 | 25.285 | 3.5194 | 78 |
| 6 | 17.542 | 5.0516 | 29 | 13 | 25.899 | 3.4373 | 36 |
| 7 | 19.195 | 4.62 | 66 | | | | |

The present invention also provides a compound of formula (IX),

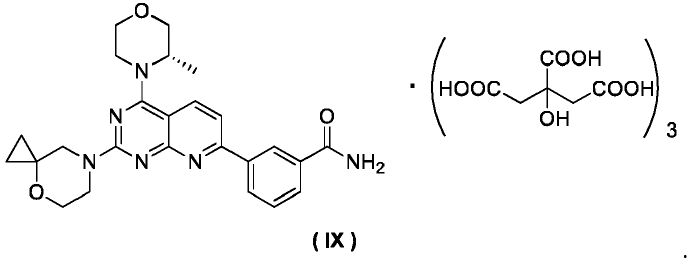

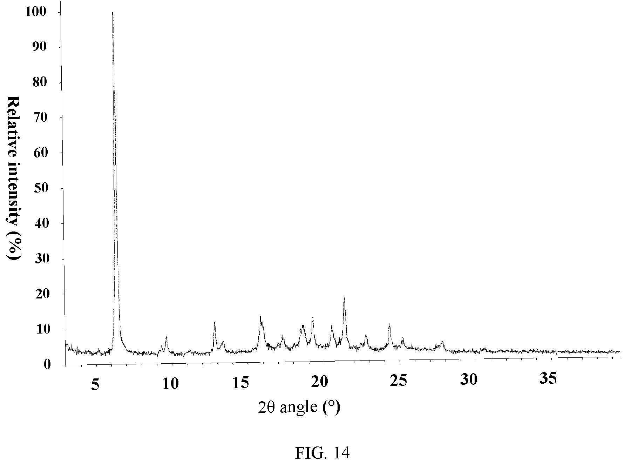

The present invention also provides a crystal form J of the compound of formula (IX), wherein the X-ray powder diffraction pattern thereof is as shown in Fig. 14.

In some embodiments of the present invention, the analytical data of the XRPD pattern of the crystal form J are shown in Table 10:

**Table 10**

| **No.** | **2θ angle (°)** | **Interplanar Distance (Å)** | **Relative Strength (%)** | **No.** | **2θ angle (°)** | **Interplanar Distance (Å)** | **Relative Strength (%)** |
|---|---|---|---|---|---|---|---|
| 1 | 6.412 | 13.7722 | 100 | 11 | 20.536 | 4.3212 | 5.8 |
| 2 | 9.314 | 9.4875 | 2.4 | 12 | 21.4 | 4.1487 | 14.2 |
| 3 | 9.667 | 9.1417 | 5.1 | 13 | 22.417 | 3.9627 | 1.5 |
| 4 | 12.824 | 6.8975 | 9.1 | 14 | 22.801 | 3.8969 | 3.8 |
| 5 | 13.375 | 6.6145 | 3.5 | 15 | 24.323 | 3.6563 | 7.4 |
| 6 | 15.997 | 5.5357 | 8.1 | 16 | 25.223 | 3.528 | 2.9 |
| 7 | 16.967 | 5.2214 | 1.5 | 17 | 27.423 | 3.2497 | 1.6 |
| 8 | 17.3 | 5.1215 | 4.2 | 18 | 27.827 | 3.2034 | 3 |
| 9 | 18.698 | 4.7416 | 6.5 | 19 | 30.608 | 2.9184 | 1.2 |
| 10 | 19.291 | 4.5974 | 8.6 | | | | |

The present invention also provides a use of the above compound or crystal form or the crystal form prepared according to the above method in the manufacture of a medicament related to the mTORC1/2 dual kinase inhibitor.

### Technical effect

The compound of formula (I) according to the present invention is tested for mTORC1/2 kinase activity, and the data show that the compound of formula (I) of the present invention has significant or even unexpected mTOR kinase inhibitory activity, which is better than the current clinical compound AZD2014.

The compound of formula (I) according to the present invention has obvious activity on the proliferation inhibition of MCF-7 and N87 cells.

PK results show that the compound of formula (I) according to the present invention has the bioavailability close to 100%, which is a good exploitable molecule for oral administration.

In the MCF-7 Xenograft tumor model, the compound of formula (I) is comparable to AZD2014, and thus the compound of formula (I) of the present invention will be a potential inhibitor for a variety of tumors.

The crystal form according to the present invention has good stability under the conditions of high temperature, high humidity and strong light.

### Definition and description

Unless otherwise stated, the following terms and phrases used herein are intended to have the following meanings. A specific phrase or term should not be considered uncertain or unclear without a special definition, but should be understood according to the common meaning. When a trade name is used herein, it is intended to refer to its corresponding commercial product or its active ingredient.

The intermediate compound according to the present invention can be prepared by a variety of synthesis methods well known to those skilled in the art, including the specific embodiments listed below, the embodiments formed by the combination of said specific embodiments with other chemical synthesis methods, and the equivalent replacement methods well known to those skilled in the art. The preferred embodiments include but are not limited to the examples of the present invention.

According to the specific embodiment in the present invention, the chemical reaction is carried out in a suitable solvent, which must be suitable for the chemical changes of the invention and the required reagents and materials. In order to obtain the compound according to the present invention, it is sometimes necessary for those skilled in the art to modify or select the synthesis step or reaction process based on the existing embodiments.

The present invention will be described in detail below through examples, which do not imply any limitations on the present invention.

All solvents used in the invention are commercially available and can be used without further purification.

The present invention applies the following abbreviations: R.T. stands for room temperature; THF stands for tetrahydrofuran; NMP stands for N-methylpyrrolidone; MeSO₃H stands for methane sulfonic acid; DME stands for ethylene glycol dimethyl ether; DCM stands for dichloromethane; Xphos stands for 2-dicyclohexylphosphine-2'4'6'-triisopropyl biphenyl; EtOAc stands for ethyl acetate; MeOH stands for methanol; 2-Me-THF stands for 2-methyltetrahydrofuran; IPA stands for isopropanol.

Compounds are named according to conventional naming principles in the art or ChemDraw^{®} software, and the commercially available compounds are named in the supplier's directory.

### X-ray powder diffraction (X-ray powder diffractometer, XRPD) method according to the invention

Instrument model: Brooke D8 advance X-ray diffractometer
Test method: about 10-20 mg sample was used for XRPD detection.
The detailed XRPD parameters were as follows:
smooth tube: Cu, kα, (λ=1.54056Ǻ)
smooth tube voltage: 40 kV, Smooth tube current: 40 mA
divergent slit: 0.60 mm
detector slit: 10.50 mm
anti-scattering slit: 7.10 mm
scan range: 4-40 deg
stepper: 0.02 deg
step: 0.12 second
rotary speed: 15 rpm

### differential scanning calorimetry (Differential Scanning Calorimeter, DSC) method according to the invention

Instrument model: TA Q2000 differential scanning calorimeter
Test method: taking the sample (~ 1 mg) and putting it into a DSC aluminum pot for test, heating the sample from 30 °C (room temperature) to 300 °C (or 350 °C) at the heating rate of 10 °C min under the condition of 50 ml/ min N₂.

### thermal gravimetric analysis (Thermal Gravimetric Analyzer, TGA) method according to the invention

Instrument model: TA Q5000IR thermal gravimetric analyzer
Test method: taking the sample (2 ~ 5 mg) and putting it into a TGA platinum pot for test, heating the sample from room temperature to 350 °C or a weight lose by 20% at the heating rate of 10 °C/ min under the condition of 25 ml/ min N₂.

### Dynamic Vapor Sorption analysis (Dynamic Vapor Sorption, DVS) method according to the invention

Instrument model: SMS DVS Advantage dynamic vapour adsorbent device
Test conditions: taking the sample (10-15 mg) and putting it into a DVS sample tray for test.
Detailed DVS parameters are as follows:
Temperature: 25 °C
Balance: dm/ dt = 0.01%/ min (minimum: 10 min, maximum: 180 min)
Drying: 120 min at 0% RH
RH(%) test step: 10%
RH(%) test step range: 0% - 90% - 0% RH (%)

The evaluation of moisture absorption is classed as follows:

| **Hygroscopic Classification** | **ΔW%** |
|---|---|
| Deliquescence | Absorbing enough water to form a liquid |
| Highly hygroscopic | ΔW% ≥ 15% |
| Hygroscopic | 15% > ΔW% ≥ 2% |
| Slightly hygroscopic | 2% > ΔW% ≥ 0.2% |
| No or little hygroscopicity | ΔW% < 0.2% |

| | |
|---|---|
| [0109] Note: Δ W% represents the hygroscopic weight gain of the test sample at 25 ± 1 °C and 80 ±2% RH. | |

### Brief description of the drawings

Figure 1 is XRPD pattern of the crystal form A of the compound of formula (I) measured by Cu-Ka radiation;
Figure 2 is DSC pattern of the crystal form A of the compound of formula (I);
Figure 3 is TGA graph of the crystal form A of the compound of formula (I);
Figure 4 is XRPD pattern of the crystal form B of the compound of formula (I) measured by Cu-Ka radiation;
Figure 5 is XRPD pattern of the crystal form C of the compound of formula (II) measured by Cu-Ka radiation;
Figure 6 is DSC pattern of the crystal form C of the compound of formula (II);
Figure 7 is TGA pattern of the crystal form C of the compound of formula (II);
Figure 8 is XRPD pattern of the crystal form D of the compound of formula (III) measured by Cu-Ka radiation;
Figure 9 is XRPD pattern of the crystal form E of the compound of formula (IV) measured by Cu-Ka radiation;
Figure 10 is XRPD pattern of the crystal form F of the compound of formula (V) measured by Cu-Ka radiation;
Figure 11 is XRPD pattern of the crystal form G of the compound of formula (VI) measured by Cu-Ka radiation;
Figure 12 is XRPD pattern of the crystal form H of the compound of formula (VII) measured by Cu-Ka radiation;
Figure 13 is XRPD pattern of the crystal form I of the compound of formula (VIII) measured by Cu-Ka radiation;
Figure 14 is XRPD pattern of the crystal form J of the compound of formula (IX) measured by Cu-Kα radiation;
Figure 15 is DVS pattern of the crystal form A of the compound of formula (I).

### Detailed description of the preferred embodiment

In order to better understand the content of the invention, the following will be further described in combination with specific examples, but the specific embodiments do not limit the content of the invention.

### Example 1: Manufacture of the compound of formula (I)

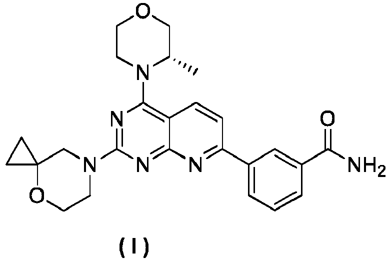

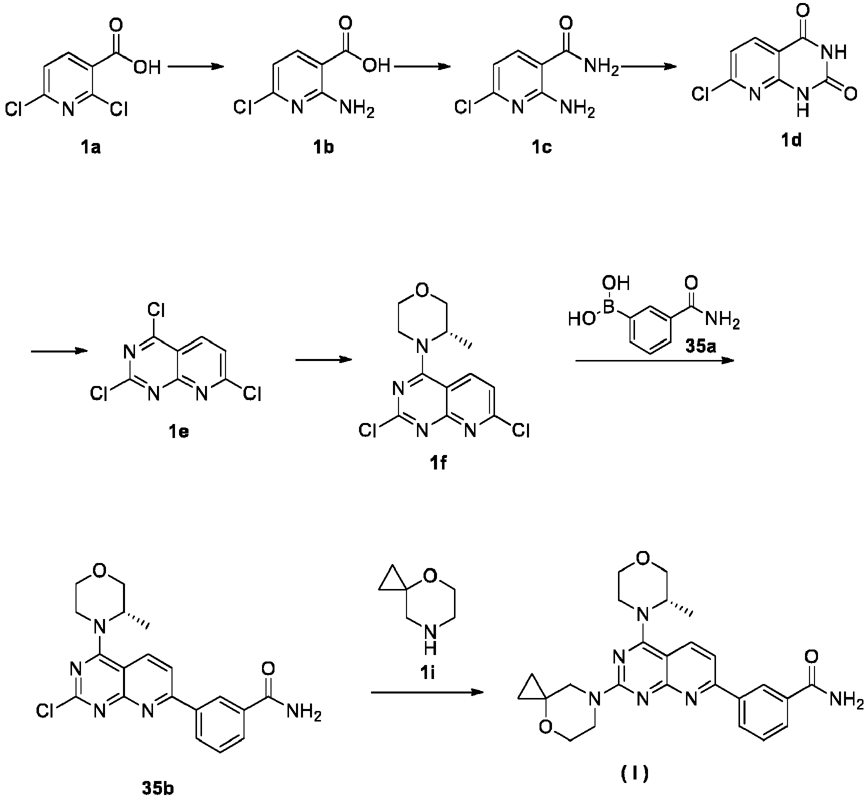

### First step

Compound 1a (20.0 g, 104 mmol, 1.00 eq) and concentrated ammonia (200 ml, 1.45 mol, 14.0 eq) were sealed in an autoclave and stirred at 130 °C for 24 hours under a pressure of about 0.9 MPa. The reaction solution was concentrated to obtain compound 1b.

MS-ESI calculated [M+H]⁺ 173 and 175, founded 173 and 175. ¹H NMR (400MHz, DMSO-d₆) δ: 8.03 (d, J=8.0 Hz, 1H), 7.56 (br s, 2H), 6.61 (d, J=8.0 Hz, 1H).

### Second step

Compound 1b (17.0 g, 98.5 mmol, 1.00 eq), ammonium chloride (10.5 g, 197 mmol, 2.00 eq), 1-hydroxybenzotriazole (13.3 g, 98.5 mmol, 1.00 eq), 1-(3-dimethylaminopropyl)-3-ethylcarbodiimide hydrochloride (18.9 g, 98.5 mmol, 1.00 eq) and diisopropyl ethylamine (38.2 g, 296 mmol, 3.00 eq) were dissolved in N, N-dimethylformamide (200.0 mL). The mixture was stirred at 20 °C for 16 hours. After the completion of the reaction, the solvent was subjected to a rotary drying under reduced pressure, water (200 mL) was added, and the resultant was extracted with ethyl acetate (200 mL × 3). The combined organic phases were dried on anhydrous sodium sulfate, filtered, and then subjected to column chromatography (1/1 petroleum ether/ ethyl acetate, Rf = 0.4) to obtain the compound. The ethyl acetate (50 mL) was beaten for ten minutes to obtain compound 1c.

¹H NMR (400MHz, DMSO-*d₆*) δ: 7.96 (d, J=8.0 Hz, 2H), 7.62 (br s, 2H), 7.40 (br s, 1H), 6.61 (d, J=8.0 Hz, 1H).

### Third step

Compound 1c (8.00 g, 46.6 mmol, 1.00 eq) and oxalyl chloride (7.1 g, 56.0 mmol, 4.9 ml, 1.00 eq) were successively added to toluene (200 mL). The mixture was stirred at 110 °C for 15 hours, cooled to room temperature, filtered and dried to obtain compound 1d.

¹H NMR (400MHz, DMSO-*d₆*) δ: 8.24 (d, J=8.0 Hz, 1H), 7.30 (d, J=8.0 Hz, 1H).

### Fourth step

Compound 1d (6.00 g, 30.4 mmol, 1.00 eq) and diisopropyl ethylamine (11.8 g, 91.1 mmol, 15.9 ml, 3.00 eq) were successively added to toluene (100 mL), and the resulting mixture was stirred at 70 °C for half an hour, cooled to room temperature and added dropwise with phosphorus oxychloride (14.0 g, 91.1 mmol, 8.5 ml, 3.00 eq). The mixture was stirred at 100 °C for 2 hours, cooled to room temperature, concentrate and subjected to column chromatography (3/ 1 petroleum ether/ ethyl acetate, Rf = 0.4) to obtain compound 1e.

¹H NMR (400MHz, DMSO-*d₆*) δ: 8.45 (d, J=8.0 Hz, 1H), 7.63 (d, J=8.3 Hz, 1H).

### Fifth step

Compound 1e (1.90 g, 8.10 mmol, 1.00 eq), (S)-2-methylmorphorphine (819 mg, 8.10 mmol, 1.00 eq) and diisopropyl ethylamine (2.09 g, 16.2 mmol, 2.83 ml, 2.00 eq) were dissolved in dichloromethane (50 mL). The resulting solution was reacted at 25 °C for 2 hours. After the completion of the reaction, the resultant was concentrated and subjected to column chromatography (3/1 petroleum ether/ ethyl acetate) to obtain compound 1f.

¹H NMR (400MHz, DMSO-*d₆*) δ: 8.47 (d, J=8.8 Hz, 1H), 7.55 (d, J=8.8 Hz, 1H), 4.71-4.72 (m, 1H), 4.12-4.09 (m, 1H), 3.92-3.91 (m, 1H), 3.84 - 3.74 (m, 1H), 3.73 - 3.64 (m, 2H), 3.54-3.53 (m, 1H), 1.46 (d, J=6.8 Hz, 3H).

### Sixth step

Compounds 1f (2 g, 6.0 mmol, 1 eq), 35a (993 mg, 6.0 mmol, 1 eq), sodium carbonate (1.9 g, 18.1 mmol, 3 eq) and dichloro(triphenylphosphine) palladium (211 mg, 301 µmol, 0.05 eq) were dissolved in anhydrous dioxane (35 mL) and water (7.0 mL). The above solution was reacted at 70 °C for 19 hours in nitrogen environment. After the completion of the reaction, the reaction solution was concentrated under reduced pressure, then added with water (15 mL) and extracted with ethyl acetate (20 mL × 3) The organic phase was treated with anhydrous sodium sulfate, collected under reduced pressure and separated by column chromatography (methanol/ ethyl acetate; Rf = 0.28) to obtain 35b.

MS-ESI calculated [M+H]⁺ 384 and 386, founded 384 and 386.

### Seventh step

Compound 35b (100 mg, 261 µmol, 1.00 eq), 1i (46.8 mg, 313 µMol, 1.20 eq) and DIPEA (101 mg, 782 µmol, 136 µL, 3.00 eq) were dissolved in DMSO (2.00 mL), and the mixed solution was reacted at 80 °C for 18 hours. After the completion of the reaction, the reaction solution was purified through high performance liquid chromatography to obtain the compound of formula (I).

MS-ESI calculated [M+H]⁺ 461, founded 461. ¹H NMR (400MHz, CDCb) δ: 8.60 (s, 1H), 8.18 (br d, J=8.0 Hz, 1H), 7.98 (d, J=8.4 Hz, 1H), 7.92 (br d, J=8.0 Hz, 1H), 7.51 (t, J=8.0 Hz, 1H), 7.43 (d, J=8.4 Hz, 1H), 6.46 (br s, 1H), 5.62 (br s, 1H), 4.30 (br s, 1H), 4.05-3.59 (m, 12H), 1.40 (d, J=6.8 Hz, 3H), 0.80-0.73 (m, 2H), 0.61 (br s, 2H).

### Example 2: Preparation of the crystal form A of compound of formula (I)

About 50.74 mg of compound of formula (I) was weighed and charged into a 2.0 mL glass vial, and thereto 200 µL of water was added to form a suspension. After adding magnetons, the above sample was placed on a magnetic oscillator (40 °C, 700 rpm) and stirred for 53 hours (away from light), then the solid was separated through centrifugation and dried overnight in a vacuum drying oven to obtain the crystal form of compound A of formula (I).

### Example 3: Preparation of the crystal form B of compound of formula (I)

About 50.63 mg of compound of formula (I) was weighed and charged into a 2.0 mL glass vial, and thereto 200 µL of methanol was added to form a suspension or solution. After adding magnetons, the above sample was placed on a magnetic oscillator (40 °C), 700 rpm) and stirred for 53 hours (away from light), then the solid was separated through centrifugation and dried overnight in a vacuum drying oven to obtain the crystal form of compound B of formula (I).

### Example 4: Preparation of the crystal form C of compound of formula (II)

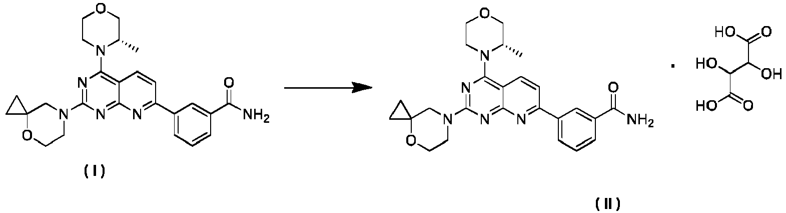

About 92 mg (0.2 mmol) of the compound was weighed and charged into a 40 mL vial, and thereto 10 mL of THF was added. The sample added with stirrer was placed on a magnetic stirrer (50 °C, 700 rpm) and stirred to obtain the suspension. The sample solution was added with tetrahydrofuran solution (553.41 µL, 0.057 mg/mL) of tartaric acid such that the molar ratio of its addition amount to the compound is 1: 1.05. The acid sample was placed on the magnetic stirrer (50 °C, 700 rpm) and stirred overnight. The mixed solution became transparent after acid addition and then was stirred overnight to form a yellow suspension. Then, the solid was separated through centrifugation and dried overnight in a vacuum drying oven to obtain the crystal form C of compound of formula (II).

### Example 5

About 92 mg (0.2 mmol) of the compound was weighed and charged into a 40 mL vial. 7 parts of the same amount of compounds were weighed and labelled as hydrochloric acid, sulfuric acid, phosphoric acid, methanesulfonic acid, maleic acid, citric acid and fumaric acid respectively. 14 mL of THF was added to the each of samples, and then the sample with stirrer was placed on a magnetic stirrer (50 °C, 700 rpm) and stirred to dissolve. The sample solution was added with an acid sample such that the molar ratio of its addition amount to the compound is 1: 1.05, and the acid sample was shown as in Table 11. The resulting sample was placed on the magnetic stirrer (50 °C, 700 rpm) and stirred overnight, during which the phenomenon was shown in Table 11. Then, the solid was separated through centrifugation and dried overnight in a vacuum drying oven to obtain the corresponding crystal form.

**Table 11**

| Acid | Acid mass volume percentage (mg/ ml) | | | Actual amount of acid added V (µL) | Phenomenon | Crystal form |
|---|---|---|---|---|---|---|
| | Actual weighed mass of solid acid (mg) volume of liquid acid (µL) | THF actual addition volume (µL) | Acid mass volume percentage (mg/ ml) | | | |
| hydrochloric acid | 100.00 | 900.00 | 0.120 | 172.44 | Yellow suspension | Crystal form D |
| sulfuric acid | 100.00 | 900.00 | 0.184 | 114.09 | Milky white suspension | Crystal form E |
| phosphoric acid | 100.00 | 900.00 | 0.169 | 143.09 | After adding acid, it started to be yellow colloid, which became yellow suspension after stirring overnight | Crystal form F |
| Methanesulfonic acid | 100.00 | 900.00 | 0.148 | 136.09 | Milky white suspension | Crystal form G |
| Maleic acid | 129.50 | 1000.00 | 0.130 | 188.01 | After adding acid and stirring overnight, the solution was clear and transparent, and precipitate was formed after volatilizing in an open vessel overnight (50 °C, 400 rpm) | Crystal form H |
| Fumaric acid | 110.60 | 2000.00 | 0.055 | 440.26 | After adding acid and stirring overnight, the solution was clear and transparent followed by volatilization in an open vessel (50 °C, 400 rpm) until it was 5 mL, and when 20 mL of anti solvent n-heptane was added, precipitate was formed | Crystal form I |
| Citric acid | 105.60 | 1000.00 | 0.106 | 419.51 | After adding acid and stirring overnight, the solution was clear and transparent followed by volatilization in an open vessel (50 °C, 400 rpm) until it was 5 mL, and when 20 mL of anti solvent MTBE was added, precipitate was formed | Crystal form J |

### Example 6: Study on hygroscopicity of the crystal form A of the compound formula (I)

### Experimental materials:

DVS Advantage dynamic vapour adsorbent device

### Experimental method:

Taking 27.36 mg of the crystal form A of the compound of formula (I) and putting it into a DVS sample disk for test.

### Experimental results:

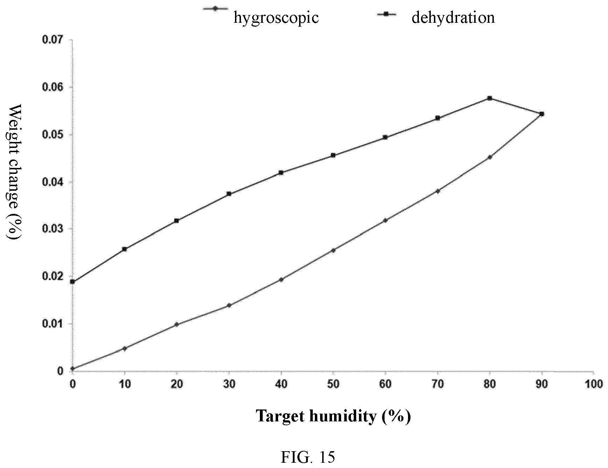

DVF graph of the crystal form A of the compound of formula (I) was shown as in Fig. 15, ΔW% < 0.2%.

### Experimental conclusion:

The crystal form A of formula (I) had a hygroscopic weight gain < 0.2% at 25.6 °C and 80% RH, without hygroscopicity.

### Example 7: Solid stability test of the crystal form of A of the compound of formula (I)

According to the guiding principles for stability test of bulk medicament and preparations (general rule 9001 of Part IV of Chinese Pharmacopoeia 2015 Edition), the crystal form A of the compound of formula (I) was investigated for its stability under long-term (25 °C 60% RH) and accelerated (30 °C / 65% RH; 40 °C / 75% RH) conditions.

51 g of the crystal form A of the compound of formula (I) was weighed and divide it into 34 parts, about 1.5 g each. Each sample was put into a double-layer LDPE Bag with each layer buckled and sealed respectively, and then the LDPE Bag was put into an aluminum foil bag and heat sealed. The samples were investigated for XRPD under the conditions of 25 °C/ 60% RH (14 bags), 30 °C 65% RH (10 bags) and 40 °C/ 75% RH (10 bags). The test results were compared with the initial test results of 0 days. The test results were shown in Table 12 below:

**Table 12 Solid stability test results of the crystal form A of the compound of formula (I)**

| Test conditions | Time point | Crystal form |
|---|---|---|
| - | Day 0 | Crystal form A |
| 25 °C/ 60% RH | 3 months | Crystal form A |
| | 6 months | Crystal form A |
| 40 °C/ 75% RH | 3 months | Crystal form A |
| | 6 months | Crystal form A |

| | | |
|---|---|---|
| [0169] Note: If the crystal form was stable under the condition of 40 °C/ 75% RH, the test will not be carried out under the condition of 30 °C/ 65% RH. | | |

Conclusion: the crystal form A of the compound of formula (I) had good stability under the conditions of high temperature, high humidity and strong light.

### Experimental example 1: evaluation of mTOR kinase inhibitory activity in vitro

### Experimental materials:

This experiment was tested on discoverx, and all materials and methods were from discoverx.

### Experiment operation:

Kinase activity analysis:
1. The labeled mTOR kinase was stably expressed in HEK-293 cells.
2. Streptavidin magnetic beads were treated with biotinylated small molecule ligands for 30 minutes at room temperature to produce an affinity resin for kinase analysis;
3. The ligand beads were blocked with excess biotin and washed with buffer (1% bovine serum albumin, 20 mL of 0.05% Tween, 1mL of dithiothreitol) to wash off unbound ligands and nonspecifically bound ligands;
4. The binding reactions of kinase ligand affinity beads, assembly and test compounds were realized in buffer (20% blocking buffer, 0.17x phosphate buffer, 0.05% Tween 20, 6 mL of dithiothreitol);
5. The test compound was dissolved in dimethyl sulfoxide;
6. All compounds used for measurement were dissolved in DMSO and then diluted directly to a concentration of 0.9%;
7. The solution was placed on 384 well polypropylene plates with a volume of 0.02 mL of each well;
8. The plate was shaked at room temperature for 1 hour;
9. The plate was washed with buffer (1x PBS, 0.05% Tween 20);
10. Affinity beads were resuspended in buffer (1x PBS, 0.05% Tween 20, 0.5 µM nonbiotin-affinity ligands) and incubated at room temperature for 30 minutes;
11. The kinase in eluent was measured for its concentration by qPCR.

The experimental results were shown in table 13.

**Table 13. Activity test results of mTORC1 and mTORC2 kinase complexes**

| Test sample | inhibitory activity of mTORC1 and mTORC2 kinase complexes Kd (nM) |
|---|---|
| Compound of formula (I) | 0.3 |

Conclusion: the compound of formula (I) had significant or even unexpected mTOR kinase inhibitory activity.

### Experimental example 2: evaluation of cell proliferation inhibitory activity

Objective: the inhibitory activity of the compound to be tested was tested on cell proliferation.

Experimental principle: Luciferase in Cell-Titer-Glo reagent uses luciferin, oxygen and ATP as reaction substrates to produce oxidized luciferin and release energy in the form of light. Since ATP is required for luciferase reaction, the total amount of light produced by the reaction is directly proportional to the total amount of ATP in response to cell viability.

### Experimental materials:

Cell lines: MCF-7 cell line (ATCC-CRL-22), HT-29 cell line (ATCC-HTB-38), OE21 (ECACC-96062201), NCI-N87 cell line (ATCC-CRL-5822)
Cell culture medium: (RPMI 1640 medium (Invitrogen # 1868546; 10% serum Invitrogen # 1804958; L-glutamine 1×), Invitrogen # 1830863; double antibody Hyclone # J170012))
Cell Titer-Glo^{®} Luminescent cell viability test kit (Promega # G7573)
384-well cell culture plate (Greiner # E15103MA)
Compound plate (LABCYTE # 0006346665)
CO₂ incubator (Thermo # 371)
Vi-cell Cell counter (Beckman Coulter)
Transferpettor (Eppendorf)
Pipette (Greiner)
Transfer liquid gun (Eppendorf)
Multifunctional enzyme labeling instrument (Envision Reader)
ECHO Liquid-handling workstation (Labcyte-ECH0555)
Experimental steps and methods:

### 2.1 Day 1:

According to the schematic diagram of cell seed plate, 384 or 96 well plate was seeded based on 1000 cells per well with a density of 25 µL per well, and the edge well was supplemented with 25 µL PBS, instead of cells.

### 2.2 Day 0:

(1) The mother liquor of the compound was 10 mM, and the compound was diluted with DMSO to obtain its initial concentration of 4 mM. The compound was added into the mother liquor plate with 9 µL per well.
(2) The compound was diluted with ECHO liquid workstation and the compound was added into the cell plate with 125 nL of the compound per well, whereinto each well in columns 2 and 23 was added with 125 nL of DMSO, and PBS well in columns 1 and 24 was added with 125 nL of DMSO.
(3) Each well in the cell plate was supplemented with 25 µL of the medium to obtain a final 50 µL per well. The compound had a concentration of 1 µM, which was diluted 3 times to obtain 10 concentrations. Duplicated wells were set left and right, and thus DMSO had a final concentration of 0.25%.

### 2.3 After the compound is added, the cell plate was centrifuged at 1000 rpm for 1 min, and then placed in an incubator under 37 °C and 5% CO₂ for 3 days.

### 2.4 Day 3:

The cell plate was taken from the incubator and balanced at room temperature for 30 minutes. 25 µL of Cell-Titer-Glo reagent was added to each well, and the plate was shaken for one minute to mix the reagent evenly followed by centrifugation at 1000 rpm for 1 minute. After 10 minutes, the plate reading was performed on PerkinElmer envision with setting the fluorescence reading time of 0.2 seconds. Test results: the test results were shown in table 14.

**Table 14. Screening test results of in vitro cell proliferation inhibitory activity of the compound of formula (I)**

| Test sample | **MCF-7 cells** | **N87 cells** |
|---|---|---|
| | **IC₅₀ (nM)** | **IC₅₀ (nM)** |
| Compound of formula (I) | 208 | 59 |

| | | |
|---|---|---|
| "ND" means not detected. | | |

Conclusion: the compound of formula (I) according to the present invention had obvious inhibitory activity on the proliferation of MCF-7 and N87 cells.

### Experimental example 3: pharmacokinetic evaluation

### Experimental method

The tested compound was mixed with 5% DMSO/ 95% 10% polyoxyethylene castor oil (Cremophor EL) followed by vortex and ultrasonic treatment to prepare 1 mg/ mL approximate clear solution, which was filtered by microporous membrane before used. BALB/ C female mice of 18 to 20 g were injected with the compound solution intravenously at a dose of 1 or 2 mg/ kg. The tested compound was mixed with 1% Tween 80, 90% polyethylene glycol 400, 90% aqueous solution followed by vortex and ultrasonic treatment to prepare 1 mg/ mL approximate clear solution, which was filtered by microporous membrane before used. BALB/ C female mice of 18 to 20 g were given the candidate compound solution orally at a dose of 2 or 10 mg/kg. Whole blood was collected for a certain time to prepare plasma. The medicament was analyzed for its concentration by LC-MS/ MS, and the pharmacokinetic parameters were calculated by Phoenix winnonlin software (pharsight company).

### Test results:

Test results were shown in Table 15 to Table 16.

**Table 15. Pharmacokinetic (PK) parameters in plasma of reference compound (AZD2014)**

| PK parameters in plasma of reference compound (AZD2014) | | | | |
|---|---|---|---|---|
| PK parameters | IV (1 mg/ kg) | IV (2 mg/ kg) | PO (2 mg/ kg) | PO (10 mg/ kg) |
| Co (nM) | 4786 | 8067 | -- | -- |
| Cₘₐₓ(nM) | -- | -- | 4370 | 16967 |
| Tₘₐₓ(h) | -- | -- | 0.500 | 0.25 |
| T_{1/2}(h) | 0.967 | 1.05 | 1.14 | 3.46 |
| V_{dss}(L/kg) | 0.677 | 0.599 | -- | -- |
| Cl(mL/min/kg) | 8.41 | 8.08 | -- | -- |
| Tₗₐₛₜ (h) | 8.00 | 8.00 | 12.0 | 24.0 |
| AUC₀₋ₗₐₛₜ (nM.h) | 4270 | 8880 | 8237 | 41443 |
| AUC_{0-inf} (nM.h) | 4281 | 8914 | 7241 | 41508 |
| MRT₀₋ₗₐₛₜ (h) | 0.268 | 1.20 | 1.95 | 2.45 |
| MRT_{0-inf} (h) | 1.88 | 1.24 | 1.95 | 2.50 |
| AUCExtra (%) | -- | 0.384 | 0.0496 | 0.156 |
| AUMC_{Extra} (%) | -- | 2.95 | 0.347 | 1.82 |
| F (%) | -- | -- | 96.2 | 93.1 |

**Table 16. PK parameters in plasma of the compound of formula (I)**

| PK parameters in plasma of the compound of formula (I) | | |
|---|---|---|
| PK parameters | IV (2 mg/ kg) | PO (10 mg/ kg) |
| Co (nM) | 8237 | -- |
| Cₘₐₓ (nM) | -- | 12850 |
| Tₘₐₓ (h) | -- | 0.25 |
| T_{1/2} (h) | 1.10 | 1.22 |
| V_{dss} (L/kg) | 0.739 | -- |
| Cl (mL/min/kg) | 7.72 | -- |
| Tₗₐₛₜ (h) | 12.0 | 12.0 |
| AUC₀₋ₗₐₛₜ (nM.h) | 9730 | 41869 |
| AUC_{0-inf} (nM.h) | 9374 | 41963 |
| MRT₀₋ₗₐₛₜ (h) | 1.59 | 3.02 |
| MRT_{0-inf} (h) | 1.60 | 3.04 |
| AUC_{Extra} (%) | 0.0469 | 0.225 |
| AUMC_{Extra} (%) | 0.400 | 1.02 |
| F (%) | -- | 89.5 |

| | | |
|---|---|---|
| Note: "--" means that no test or data was obtained | | |

Test conclusion: the compound of formula (I) showed the same or even better pharmacokinetic properties as the reference compound; the compound of formula (I) according to the present invention had a bioavailability close to 100%, which was a good exploitable molecule for oral administration.

### Experiment 4: in vivo pharmacodynamic study of subcutaneous xenografts of human breast cancer MCF-7 cell in nude BALB/c mice model

Experimental objective: to study the in vivo efficacy of the compound to be tested on subcutaneous xenografts of human breast cancer MCF-7 in nude BALB/c mice model.

Experimental animals: female BALB/ C nude mice, 6-8 weeks old, weighing 18-22 g; Supplier: Shanghai Xipur Bikai Experimental Animal Co., Ltd.

### Experimental methods and steps:

### 4.1 cell culture

Human breast cancer MCF-7 cells (ECACC, item number: 86012803) were cultured in monolayer in vitro. The culture conditions were EMEM (EBSS) + 2 mM glutamic acid + 1% Non Essential Amino Acids (NEAA) medium plus 10% fetal bovine serum, 100 U/mL penicillin and 100 µg/mL streptomycin, incubated in 5% CO₂ incubator at 37 °C. Cells were passaged through routine digestion using Trypsin EDTA twice a week. When the cell saturation was 80%-90% and the number reached the requirements, cells were collected, counted and inoculated.

### 4.2 Tumor cell inoculation (tumor inoculation)

Estrogen tablets (0.18 mg) were inoculated on the left back of each mouse 3 days before cell inoculation. 0.2 mL (1 × 10⁷) of MCF-7 cells (with matrix glue and volume ratio of 1:1) was subcutaneously inoculated on the right back of each mouse. The medicament was administered in groups when the average tumor volume reached 142 mm³.

### 4.3 Formulation of the test compound

The test compound was formulated into clear solutions of 0.75 mg/ mL, 1.5 mg/ mL and 3 mg/ mL respectively, and the solvent was 5% DMSO + 30% polyethylene glycol 300 + 65% water.

### 4.4 Tumor measurement and experimental indicators

The experimental index is used for investigating whether tumor growth is inhibited, delayed or cured. The tumor diameter was measured with a vernier caliper twice a week. The calculation formula of tumor volume is: V = 0.5a ×b², wherein a and b represent the long diameter and short diameter of the tumor, respectively.

The antitumor effect of the compound was evaluated by TGI (%) or relative tumor proliferation rate T/ C(%). TGI (%), reflecting tumor growth inhibition rate. TGI (%) was calculated: TGI (%) = [1 - (average tumor volume at the end of administration in a certain treatment group - average tumor volume at the beginning of administration in the treatment group) / (average tumor volume at the end of treatment in the solvent control group - average tumor volume at the beginning of treatment in the solvent control group)] × control group.

Relative tumor proliferation rate T/ C (%): the calculation formula is as follows: T/ C% = TRTV / CRTV (TRTV: RTV in treatment group; CRTV: RTV in negative control group). The relative tumor volume (RTV) is calculated according to the results of tumor measurement. The calculation formula is RTV=Vₜ/V₀, where Vo is the average tumor volume measured in group administration (i.e. do), Vt is the average tumor volume measured in a certain measurement, and data on the same day were taken for TRTV and CRTV.

After the experiment, the tumor weight will be detected and the T/ C weight percentage will be calculated. T weight and C weight represent the tumor weight of the administration group and the solvent control group respectively.

### 4.5 Statistical analysis

Statistical analysis included the mean and standard error (SEM) of tumor volume at each time point of each group. The treatment group showed the best treatment effect on the 21 st day after administration at the end of the trial. Therefore, statistical analysis was conducted based on these data to evaluate the difference between the groups. The comparison between the two groups was analyzed by T-test, and the comparison between the three or more groups was analyzed by one-way ANOVA. If there was a significant difference in F value, the Games-Howell method was used for test. If there was no significant difference in F value, Dunnet (2-sided) method was used for analysis. All data were analyzed with SPSS 17.0. If P < 0.05, the difference was considered significant.

### 4.6 The experimental results were shown in Table 17.

**Table 17. Anti-tumor effect of the compound on subcutaneous xenografts of human breast cancer MCF-7 cell model**

| **Group** | **Tumor volume (mm³)^{a} (day 0)** | **Tumor volume (mm³)^{a} (day 27)** | **RTV (day 27)** | **T/C^{b} (%)** | **TGI^{b} (%)** |
|---|---|---|---|---|---|
| Solvent | 142 ±7 | 588 ±75 | 4.17 ± 0.52 | -- | -- |
| AZD2014 (7.5 mg/ kg) | 142 ±9 | 315 ±65 | 2.25 ± 0.47 | 53.95 | 61.10 |
| AZD2014 (15 mg/ kg) | 141 ±8 | 121 ±41 | 0.85 ± 0.26 | 20.36 | 104.37 |
| Compound of formula (I) (15 mg/kg) | 142 ±11 | 323 ±25 | 2.41 ± 0.36 | 57.84 | 59.27 |

| | | | | | |
|---|---|---|---|---|---|
| [0242] Note: [0243] "--" does not need to be calculated [0244] a. Mean ± SEM. [0245] b. Tumor growth inhibition was calculated by T/ C and TGI (TGI (%) = [1-(T₂₁-T₀)/(V₂₁-V₀)]×100). | | | | | |

Experimental conclusion: in the MCF-7 Xenograft tumor model, the efficacy of the compound of formula (I) was equivalent to that of AZD2014.

## Claims

1. A crystal form A of the compound of formula (I), wherein the X-ray powder diffraction pattern thereof has characteristic diffraction peaks at the following 2θ angles: 16.355 ±0.200°, 19.847 ±0.200°, and 21.319 ±0.200°

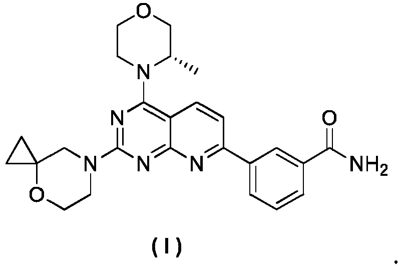

2. The crystal form A of the compound of formula (I) according to claim 1, wherein the X-ray powder diffraction pattern thereof has characteristic diffraction peaks at the following 2θ angles: 8.169 ±0.20010.986 ±0.200° 13.394 ±0.200° 16.355 ± 0.200°, 17.084 ±0.200°, 19.847 ±0.200°, 21.319 ±0.200°, and 22.154 ±0.200°.

3. The crystal form A of the compound of formula (I) according to claim 2, wherein the X-ray powder diffraction pattern thereof has characteristic diffraction peaks at the following 2θ angles: 8.169 ±0.200°, 9.903 ±0.200°, 10.986 ±0.200°, 13.394 ± 0.200°, 13.931 ±0.200° 15.330 ±0.200°, 16.355 ±0.200°, 17.084 ±0.200° 19.847 ± 0.200°, 21.319 ±0.200° 22.154 ±0.200°, and 23.475 ±0.200°

4. The crystal form A of the compound of formula (I) according to claim 3, wherein the X-ray powder diffraction pattern thereof has characteristic diffraction peaks at the following 2θ angles: 8.169°, 9.119°, 9.551°, 9.903°, 10.986°, 12.525°, 13.394°, 13.931°, 15.330°, 15.589°, 15.923°, 16.355°, 16.743°, 17.084°, 17.815°, 18.268°, 19.149°, 19.414°, 19.847°, 20.273°, 21.319°, 22.154°, 23.475°, 23.953°, 24.710°, 25.134°, 25.805°, 26.054°, 26.232°, 26.772°, 28.048°, 29.254°, 29.469°, 29.958°, 31.617 °, 31.838 °, 33.074 °, 33.924 °, 34.281°, 36.061°, 36.520 °, and 38.052 °.

5. The crystal form A of the compound of formula (I) according to claim 4, wherein the XRPD pattern thereof is shown in Fig. 1.

6. The crystal form A of the compound of formula (I) according to any one of claims 1-5, wherein the differential scanning calorimetry curve thereof has an endothermic peak at 221.62 ±3.00 °C.

7. The crystal form A of the compound of formula (I) according to claim 6, wherein the DSC pattern thereof is shown in Fig. 2.

8. The crystal form A of the compound of formula (I) according to any one of claims 1-5, wherein the thermal gravimetric analysis curve thereof has a weight loss of 0.08225% at 122.98 ±3.00 °C, and a further weight loss of 0.4156% at 262.62 ±3.00 °C.

9. The crystal form A of the compound of formula (I) according to claim 8, wherein the TGA graph thereof is shown in Fig. 3.

10. A crystal form B of the compound of formula (I), wherein the X-ray powder diffraction pattern thereof has characteristic diffraction peaks at the following 2θ angles: 6.295 ±0.200°, 7.851 ±0.200°, 11.324 ±0.200°, and 16.351 ±0.200 °.

11. The crystal form B of the compound of formula (I) according to claim 10, wherein the X-ray powder diffraction pattern thereof has characteristic diffraction peaks at the following 2θ angles: 6.295 ±0.200°, 7.851 ±0.200°, 8.621 ±0.200°, 11.324 ± 0.200°, 14.184 ±0.200°, 15.287 ±0.200°, 16.351 ±0.200°, and 26.114 ±0.200°.

12. The crystal form B of the compound of formula (I) according to claim 11, wherein the X-ray powder diffraction pattern thereof has characteristic diffraction peaks at the following 2θ angles: 6.295 ±0.200°, 7.851 ±0.200°, 8.621 ±0.200°, 11.324 ± 0.200°, 14.184 ±0.200°, 15.287 ±0.200°, 16.351 ±0.200°, 17.087 ±0.200°, 18.563 ± 0.200°, 19.531 ±0.200°, 20.791 ±0.200°, and 26.114 ±0.200°.

13. The crystal form B of the compound of formula (I) according to claim 12, wherein the X-ray powder diffraction pattern thereof has characteristic diffraction peaks at the following 2θ angles: 6.295°, 7.851°, 8.166°, 8.621°, 9.109°, 9.528°, 9.897°, 10.985°, 11.324°, 11.973°, 12.586°, 12.876°, 13.391°, 13.928°, 14.184°, 15.287°, 15.879°, 16.351°, 16.590°, 17.087°, 17.276°, 18.563°, 18.933°, 19.531°, 19.827°, 20.791°, 21.285°, 22.136°, 22.585°, 23.489°, 23.650°, 24.151°, 24.970°, 25.328°, 25.900°, 26.114°, 26.731°, 28.109°, 28.659°, 29.551°, 29.908°, 31.539°, 36.554°, and 38.644°.

14. The crystal form B of the compound of formula (I) according to claim 13, wherein the XRPD pattern thereof is shown in Fig. 4.

15. Compound of formula (II),

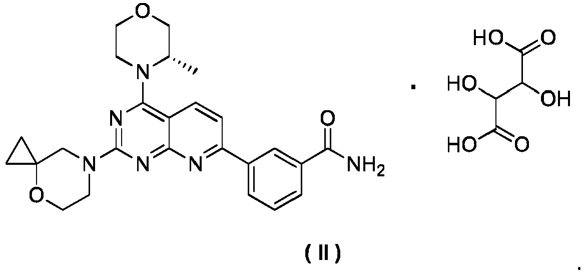

16. A crystal form C of the compound of formula (II), wherein the X-ray powder diffraction pattern thereof has characteristic diffraction peaks at the following 2θ angles: 5.802 ±0.200°, 7.991 ±0.200°, 11.618 ±0.200°, and 16.567 ±0.200°.

17. The crystal form C of the compound of formula (II) according to claim 16, wherein the X-ray powder diffraction pattern thereof has characteristic diffraction peaks at the following 2θ angles: 5.802 ±0.200°, 7.991 ±0.200°, 11.618 ±0.200 °, 13.138 ± 0.200 °, 15.487 ±0.200°, 15.959 ±0.200°, 16.567 ±0.200 °, and 18.268 ±0.200°.

18. The crystal form C of the compound of formula (II) according to claim 17, wherein the X-ray powder diffraction pattern thereof has characteristic diffraction peaks at the following 2θ angles: 5.802 ± 0.200°, 7.991 ±0.200°, 11.618 ±0.200°, 13.138 ± 0.200°, 15.487 ±0.200° 15.959 ±0.200°, 16.567 ±0.200°, 18.268 ±0.200° 18.802 ± 0.200°, 19.505 ±0.200°, 22.309 ±0.200°, and 23.633 ±0.200°.

19. The crystal form C of the compound of formula (II) according to claim 18, wherein the X-ray powder diffraction pattern thereof has characteristic diffraction peaks at the following 2θ angles: 5.802°, 7.991°, 11.127°, 11.618°, 13.138°, 14.219°, 14.637°, 15.487°, 15.959°, 16.567°, 18.268°, 18.802°, 19.098°, 19.505°, 19.881°, 20.343°, 21.061°, 21.340°, 21.977°, 22.309°, 22.504°, 23.298°, 23.633°, 24.403°, 24.954°, 25.286°, 25.557°, 26.176°, 26.986°, 28.618°, 29.472°, 29.805°, 31.796°, 32.052°, 32.666°, 33.433°, 34.423°, 34.836°, 38.939°, and 39.312°.

20. The crystal form C of the compound of formula (II) according to claim 19, wherein the XRPD pattern thereof is as shown in Fig. 5.

21. The crystal form C of the compound of formula (II) according to any one of claims 16-20, wherein the differential scanning calorimetry curve thereof has an endothermic peak at 229.91 ±3.00 °C.

22. The crystal form C of the compound of formula (II) according to claim 21, wherein the DSC pattern thereof is shown in Fig. 6.

23. The crystal form C of the compound of formula (II) according to any one of claims 16-20, wherein the thermal gravimetric analysis curve thereof has a weight loss of 0.1862% at 143.66 ±3.00 °C.

24. The crystal form C of the compound of formula (II) according to claim 23, wherein the TGA pattern thereof is as shown in Fig. 7.

25. A method for preparing the crystal form A of compound of formula (I), comprising:
(a) adding the compound of formula (I) to a solvent;
(b) stirring at 30-50 °C for 45-55 hours;
(c) obtaining a solid by centrifugation as the crystal form A of compound of formula (I);
wherein said solvent is water, acetone, acetonitrile, tetrahydrofuran, methyl t-butyl ether or ethyl acetate.

26. A use of the compound according to claim 15 and the crystal form according to any one of claims 1-14 and claims 16-24 in manufacture of a medicament related to the mTORC1/ 2 dual kinase inhibitor.
